# EUROPÄISCHE PATENTSCHRIFT

(11) **EP 2 324 349 B1**
(45) Veröffentlichungstag und Bekanntmachung des Hinweises auf die Patenterteilung: **30.11.2016**
(21) Anmeldenummer: 08801819.7
(22) Anmeldetag: 03.09.2008
(51) Int. Cl.: G01N 33/00

(54) **VERFAHREN UND VORRICHTUNG ZUR MESSWERTERFASSUNG UND MESSWERTANZEIGE**
METHOD AND APPARATUS FOR CAPTURING MEASUREMENT VALUES AND DISPLAYING MEASUREMENT VALUES
PROCÉDÉ ET DISPOSITIF DE SAISIE DE VALEURS DE MESURE ET D'AFFICHAGE DE VALEURS DE MESURE

(43) Veröffentlichungstag der Anmeldung: 25.05.2011
(73) Patentinhaber: Testo AG, 79853 Lenzkirch (DE)
(72) Erfinder: HOYER, Knut, 78056 Schwenningen (DE); KAUFMANN, Andreas, 79199 Kirchzarten (DE); MÜNCH, Reinhold, 79111 Freiburg (DE); SPRINGMANN, Thomas, 79199 Kirchzarten (DE)
(74) Vertreter: Kunst, Manuel Nikolaus Johannes
(86) Internationale Anmeldenummer: PCT/EP2008/007190
(87) Internationale Veröffentlichungsnummer: WO 2010/025745

(56) Entgegenhaltungen:
- EP-A- 1 715 338
- US-A- 3 979 589
- US-A- 4 560 873
- US-A- 4 829 183
- US-A- 5 418 366
- US-B1- 6 174 421
- US-B1- 6 319 377

## Beschreibung

Die Erfindung betrifft ein Verfahren zur Bestimmung einer NOₓ-Konzentration in einem Messgas, wobei ein Sensorsignal eines für NOₓ sensitiven und mit dem Messgas in Kontakt gebrachten Gassensors erfasst wird und aus dem Sensorsignal ein Messwert für die NOₓ-Konzentration bestimmt wird, und ein Gerät zur Ausführung dieses Verfahrens.

Derartige Verfahren sind bereits bekannt, wobei als Gassensoren bevorzugt Chemilumineszenz-Detektoren (CLD) Verwendung finden. Beispielsweise werden derartige Verfahren verwendet, um gesetzliche Grenzwerte zu überprüfen, beispielsweise zur Bestimmung der spezifischen Stickoxid-Emission als Abgas-Kennzahl eines Verbrennungsmotors, allerdings finden diese Verfahren auch in anderen Applikationen ihre Anwendung.

Häufig werden aus Gründen des Klimaschutzes oder aus sonstigen politischen Gründen gesetzliche Grenzwerte für den Stickoxid-Gehalt in einem Messgas, beispielsweise dem Abgas eines Verbrennungsmotors, eingerichtet, bei deren Nichteinhaltung Sanktionen verhängt werden können. Derartige Vorgaben beziehen sich häufig auf ein konkret vorgegebenes Messverfahren, um Rechtsunsicherheiten auszuschließen.

Aus der US 4,829,183 A ist ein IR-Gasanalysator mit einer Probenkammer für gekühltes Probengas und einer Probenkammer für ungekühltes Probengas bekannt, wobei hinter den Probenkammern jeweils Filter mit IR-Detektoren für die Absorptionslinien der unterschiedlichen Komponenten des Probengases angeordnet sind, wobei das Ergebnis der NOₓ-Messung durch ein Signal des Rest-Wasserdampfs korrigiert wird.

Aus der US 5,418,366 A ist ein IR-basierter Stickoxid-Sensors mit einer Wasserdampfkompensation bekannt.

Aus der US 3,979,589 A sind ein Verfahren und ein System für die IR-Analyse eines Gases bekannt, bei welchem ein Verarbeitungsschaltkreis zur elektrischen Kompensation von Absorptionsband-Indifferenzen eingerichtet ist.

Aus der US 6,174,421 B1 ist ein Sensor zur Bestimmung der Konzentration von oxidierbaren Elementen in einem Gasgemisch bekannt, bei welchem zwei oder mehr Messelektroden mit unterschiedlichen katalytischen Aktivitäten vorgesehen sind, wobei Querempfindlichkeiten einer ersten Messelektrode durch Verwendung der Signale der zusätzlichen Messelektroden vollständig oder zumindest teilweise dadurch kompensiert werden, dass die Empfindlichkeiten dieser zusätzlichen Messelektroden an die interferierenden Gaskomponenten angepasst werden.

Aus der EP 1 715 338 A2 ist ein Abgaskomponenten-Analysator bekannt, bei welchem ein Chemilumineszenz-Stickoxid-Analysator zur Messung der Konzentration von NOₓ vorgesehen ist, wobei der tatsächliche Messwert mittels eines Korrekturwertes aus der CO₂-Konzentration und der H₂O-Konzentration korrigiert wird.

Aus der US 6,319,377 B1 ist ein Stickoxid-Sensor bekannt, bei welchem die Mess-Konzentration von Stickoxiden zusätzlich zur Steuerung einer Sauerstoff-Konzentration mittels einer Sauerstoff-Pumpe durch Oxidierung oder Reduzierung der Stickoxide an den Elektrodenoberflächen erhöht wird.

Aus P. Glatz und M. Kutter, "Very Low Level NO/NOx Measurement - Unlimited?", SGA-BULLETIN, Nr. 27, Sept.-Nov. 1999, S.1 bis 8, ist ein Verfahren der eingangs genannten Art bekannt, wobei als Gassensor ein Chemilumineszenz-Detektor vorgeschlagen wird. Zur Verbesserung der Lichtausbeute und unterdrückung des sogenannten Quenchings wird der Druck in der Reaktionskammer erniedrigt und permanent mit einem ausgeklügelten Elektronik-System überwacht.

Der Erfindung liegt die Aufgabe zugrunde, ein verbessertes Verfahren zur Bestimmung einer NOₓ-Konzentration in einem Messgas zu schaffen, welches zur Kontrolle der Einhaltung von gesetzlichen oder sonstigen Vorgaben geeignet ist.

Zur Lösung dieser Aufgabe sind bei einem Verfahren der eingangs genannten Art die Merkmale des Anspruchs 1 vorgesehen.

Alternativ sind zur Lösung dieser Aufgabe bei einem Verfahren der eingangs genannten Art die Merkmale des Anspruchs 2 vorgesehen.

Insbesondere ist vorgesehen, dass ein Messwert für die Konzentration eines zweiten Bestandteils im Messgas ermittelt wird und dass aus dem Messwert für die Konzentration des zweiten Bestandteils im Messgas ein Zahlenwert für die NOₓ-Konzentration im Messgas bestimmt wird. Das erfindungsgemäße Verfahren bietet den Vorteil, dass durch die Ermittlung der Konzentration eines zweiten Bestandteils im Messgas ein gesetzlich vorgeschriebener Gassensor, und damit ein gesetzlich vorgeschriebenes Messverfahren oder ein als Referenz etablierter Gassensor-Typ beziehungsweise ein etabliertes Referenz-Messverfahren simulierbar sind, beziehungsweise systematische Messfehler eines vorgeschriebenen Messverfahrens für sonstige Messungen korrigierbar sind, wobei die Brauchbarkeit des verwendeten Verfahrens für Aussagen über die Einhaltung eines vorgegebenen Grenzwerts oder für den Vergleich mit anderen Zahlenwerten nicht beeinträchtigt beziehungsweise ermöglicht wird. Das erfindungsgemäße Verfahren ist auch zur Motoreneinstellung vorteilhaft verwendbar, bei welchem ein Monteur ein vergleichbares Messgerät verwenden soll, das einen Vergleich mit anderweitig aufgenommenen Messwerten bzw. mit anderen Messverfahren erlaubt.

Gemäß einer Ausgestaltung der Erfindung ist vorgesehen, dass der zweite Bestandteil Sauerstoff ist. Versuche haben ergeben, dass bei den häufig eingesetzten Chemilumineszenz-Detektoren ein systematischer Messfehler bei der Bestimmung der Konzentration von NOₓ auftritt, der beispielsweise durch die Anwesenheit von O₂ hervorgerufen werden kann. Es hat sich gezeigt, dass das bei niedrigen NOₓ-Konzentrationen bewährte CLD-Verfahren bei hohen O₂- und NOₓ-Konzentrationen systematische Abweichungen aufweist. Das erfindungsgemäße Verfahren ermittelt somit einen Messwert für die NOₓ-Konzentration und einen Zahlenwert für die NOₓ-Konzentration, wobei der Zahlenwert als korrigierter Messwert oder als Differenz oder Faktor zu einem korrigierten Messwert verwendbar ist. Alternativ oder zusätzlich kann als zweiter Bestandteil eine Sauerstoffverbindung, beispielsweise CO₂, verwendet werden. Hierbei ist beispielsweise der O₂-Anteil indirekt über den CO₂-Anteil und den λ-Wert des Messgases bestimmbar, oder es wird der direkte Einfluss von CO₂ auf die mit einem vorgegeben Messverfahren ermittelte NOₓ-Konzentration berücksichtigt.

Gemäß einer Ausgestaltung der Erfindung kann vorgesehen sein, dass der Messwert und der Zahlenwert für die NOₓ-Konzentration im Messgas angezeigt werden. Somit kann der Verwender des Verfahrens selbst entscheiden, welchen der beiden angezeigten Werte er für seinen konkreten Gebrauch weiterverwenden will, wobei der Zahlenwert ein korrigierter Messwert oder ein Differenzwert, der auf den Messwert aufzuschlagen oder von diesem abzuziehen ist, oder ein Faktor, mit dem der Messwert zu multiplizieren ist, sein kann.

Zur Überwachung von Vorgaben für Verbrennungsmotoren kann vorgesehen sein, dass das Messgas aus den Abgasen eines Verbrennungsmotors, beispielsweise eines Diesel-Motors, entnommen wird.

Die Ermittlung der Konzentration des zweiten Bestandteils im Messgas kann durch ein separates Analyseverfahren oder durch anderweitige Eingabe der betreffenden Werte erfolgen, wobei der zweite Bestandteil Sauerstoff ist. Für eine automatisierte Durchführung des erfindungsgemäßen Verfahrens ist jedoch vorteilhaft, wenn zur Ermittlung der Konzentration des weiteren Bestandteils im Messgas ein zweiter Gassensor vorgesehen ist.

Zur Ermittlung des Messwerts für die NOₓ-Konzentration aus dem Sensorsignal des Gassensors ist vorgesehen, dass der Messwert für die NOₓ-Konzentration im Messgas anhand einer hinterlegten ersten Kennlinie aus dem Sensorsignal des ersten Gassensors bestimmt wird und der Zahlenwert für die NOₓ-Konzentration in Messgas anhand einer hinterlegten zweiten Kennlinie aus dem Sensorsignal des ersten Gassensors und des zweiten Gassensors bestimmt wird, wobei die erste Kennlinie die Kennlinie eines NOₓ-sensitiven Infrarot-Sensors, UV-Sensors, elektrochemischen Sensors oder Halbleitersensors ist und die zweite Kennlinie die Abweichungen der Kennlinie eines NOₓ-sensitiven Chemilumineszenz-Detektors von der ersten Kennlinie bei unterschiedlichen Werten der Konzentration des weiteren Bestandteils im Messgas abbildet.

Eine besonders einfache Bestimmung der Messwerte beziehungsweise Zahlenwerte ergibt sich, wenn die erste Kennlinie und/oder die zweite Kennlinie durch Interpolation oder Extrapolation von Kalibrier- oder Stützpunkten jeweils einer Kennlinienkurve gebildet ist/sind. Somit wird vorteilhaft erreicht, dass zur Einrichtung des erfindungsgemäßen Verfahrens die Aufnahme nur einer geringen Anzahl von Kalibrierpunkten erforderlich ist. Die Kennlinien werden vorzugsweise durch Kalibrier- und/oder Vergleichsmessungen definiert und/oder ermittelt.

Zur Erzielung einer verbesserten Messgenauigkeit kann vorgesehen sein, dass die erste Kennlinie und/oder die zweite Kennlinie als Datenreihe, also mit einer Vielzahl von Messpunkten, hinterlegt ist/sind. Dies ist insbesondere dann vorteilhaft, wenn bei einer Interpolation oder Extrapolation aufgrund der spezifischen Eigenschaften des verwendeten Gassensortyps unerwünschte Abweichungen und/oder systematische Fehler zu erwarten sind.

Gemäß einem vorteilhaften Ausführungsbeispiel der Erfindung kann vorgesehen sein, dass die erste Kennlinie die Kennlinie eines elektrochemischen Sensors, eines Infrarot-Sensors, eines UV-Sensors oder eines Halbleitersensors ist, und dass die zweite Kennlinie die Abweichungen eines Chemilumineszenz-Detektors von der ersten Kennlinie bei unterschiedlichen Werten der Konzentration des weiteren Bestandteils im Messgas abbildet. Versuche haben ergeben, dass Infrarot-Sensoren, UV-Sensoren oder elektrochemische Sensoren, die vorzugsweise NOₓ-sensitiv ausgebildet sind, bei einem hohem O₂-Anteil im Messgas ein von den häufig vorgeschriebenen Chemilumineszenz-Detektoren abweichendes Messverhalten zeigen, bei welchem eine unerwünschte systematische Fehlerquelle eliminiert oder zumindest vermindert ist. Statt der elektrochemischen Sensoren sind auch Halbleitersensoren einsetzbar.

Gemäß einem weiteren vorteilhaften Ausführungsbeispiel kann vorgesehen sein, dass die erste Kennlinie die Kennlinie eines Chemilumineszenz-Detektors ist und dass die zweite Kennlinie die Abweichungen der Kennlinie eines elektrochemischen Sensors, eines Infrarot-Sensors, UV-Sensors oder Halbleitersensors von der ersten Kennlinie bei unterschiedlichen Werten der Konzentration des weiteren Bestandteils im Messgas abbildet. Somit wird vorteilhaft erreicht, dass selbst bei Einsatz eines häufig gesetzlich oder aus sonstigen Gründen vorgeschriebenen Chemilumineszenz-Detektors mit dem erfindungsgemäßen Verfahren korrigierte Messwerte für die NOₓ-Konzentration bereitstellbar sind, die den beschriebenen systematischen Fehler berücksichtigen.

Der Infrarot-Sensor ist vorzugsweise als nicht-dispersiver IR-Sensor ausgebildet. Der CLD-Sensor ist vorzugsweise NOₓ-sensitiv ausgebildet.

Eine besonders vorteilhafte Ausgestaltung ergibt sich, wenn für die erste Kennlinie bzw. für die zweite Kennlinie ein elektrochemischer Sensor verwendet wird.

Für eine vereinfachte Überwachung der gesetzlichen Vorgaben kann vorgesehen sein, dass die Über- und/oder Unterschreitung von Grenzwerten für Messwert und/oder Zahlenwert für die NOₓ-Konzentration im Messgas getrennt signalisiert wird. Die Signalisierung kann optisch, akustisch oder per Niederschrift in ein Protokoll oder auf sonstigem Wege erfolgen. Somit kann sich der Benutzer vorteilhaft auf die um systematische Fehler bereinigten Angaben zur NOₓ-Konzentration beschränken, während durch das erfindungsgemäße Verfahren sichergestellt wird, dass die Über- und/oder Unterschreitung von gesetzlich oder aus sonstigen Gründen vorgegebenen Grenzwerten unabhängig von dieser Beschränkung signalisiert wird.

Beispielsweise kann bei einer Ausgestaltung des erfindungsgemäßen Verfahrens vorgesehen sein, dass die Differenz zwischen Messwert und Zahlenwert für die NOₓ-Konzentration im Messgas mit zunehmender Konzentration des zweiten Bestandteils im Messgas zunimmt oder abnimmt.

Ein weiter verbessertes Verfahren ergibt sich dadurch, dass wenigstens ein weiterer Mess- und/oder Kennwert für die Konzentration eines weiteren Bestandteils im Messgas ermittelt und/oder eingegeben wird und dass aus dem weiteren Mess- und/oder Kennwert ein Korrekturwert für die NOₓ-Konzentration im Messgas bestimmt wird. Beispielsweise kann vorgesehen sein, dass durch die Konzentration des weiteren Bestandteils im Messgas der Feuchtigkeitsgehalt im Messgas bestimmt wird, insbesondere dass der weitere Bestandteil Wasser beziehungsweise Wasserdampf ist. Alternativ oder zusätzlich kann vorgesehen sein, dass der weitere Bestandteil CO₂ ist. Durch die Berücksichtigung weiterer Bestandteile werden systematische Fehler durch die Anwesenheit weiterer Bestandteile noch besser berücksichtigt, oder es wird der Einfluss des Feuchtigkeitsgehalts auf den Messwert berücksichtigt. Die Bestimmung des wenigstens einen Bestandteils erfolgt beispielsweise durch einen entsprechend ausgebildeten Gassensor.

Zur weiteren Verbesserung des Verfahrens kann vorgesehen sein, dass die Bestimmung des Zahlenwertes für die NOₓ-Konzentration im Messgas aus dem Messwert für die Konzentration des zweiten Bestandteils und/oder des weiteren Bestandteils in Abhängigkeit vom Feuchtigkeitsgehalt des Messgases erfolgt. Der Feuchtigkeitsgehalt des Messgases kann hierbei beispielsweise durch Eingabe eines Zahlenwertes und/oder durch Eingabe eines Taupunktes im Messgas und/oder durch Messung am Messgas, beispielsweise mit einer ZrO₂-Sonde, erfolgen. Versuche haben ergeben, dass der Einfluss des Feuchtigkeitsgehalts im Messgas erheblich ist, wenn erhöhte Anforderungen an die Messgenauigkeit gestellt werden, da sich in dem im Messgas enthaltenen Wasser Sauerstoff lösen kann, wodurch eine Korrektur von Querempfindlichkeiten erst Recht vorteilhaft ist. Für eine einfache Realisierung kann vorgesehen sein, dass der Feuchtigkeitsgehalt des Messgases lediglich in Stufen, beispielsweise in zwei, drei oder mehr als drei Stufen, berücksichtigt wird.

Bei Verwendung einer dem Gassensor vorgeschalteten Gastrocknungseinheit, beispielsweise eines Gaskühlers, ist die Berücksichtigung des Feuchtigkeitsgehalts im Messgas erforderlich, wenn die NOₓ-Konzentration für feuchtes Messgas ermittelt werden soll. Umgekehrt kann der Feuchtigkeitsgehalt verwendet werden, um bei einer Messung ohne Gastrocknung eine NOₓ-Konzentration für getrocknetes Messgas zu ermitteln bzw. zu errechnen.

Wenn es sich bei dem Messgas um das Abgas eines Verbrennungsmotors handelt, kann der Feuchtigkeitsgehalt auch aus den Parametern der dem Verbrennungsmotor zugeführten chemischen Substanzen, insbesondere des Kraftstoffs und/oder der Luft, und der Verbrennungsprodukte errechnet werden. Alternativ kann auch hierbei der Feuchtigkeitsgehalt direkt gemessen werden.

Zur Berücksichtigung des Feuchtigkeitsgehalts des Messgases kann hierbei vorgesehen sein, dass bei den zugeführten chemischen Substanzen über eine Elementaranalyse Elementarbestandteile, insbesondere H und/oder C, bestimmt werden und/oder dass der Feuchtigkeitsgehalt des Messgases berechnet wird, insbesondere aus den bestimmten Elementarbestandteilen. Vorzugsweise werden in der Elementaranalyse Konzentrationen der Elementarbestandteile bestimmt, aus denen sich der Feuchtigkeitsgehalt ermitteln lässt. Alternativ oder zusätzlich kann der Feuchtigkeitsgehalt der dem Verbrennungsmotor zugeführten Luft bestimmt werden.

Zur Lösung der Aufgabe und insbesondere zur Durchführung des erfindungsgemäßen Verfahrens sind bei einem Gerät der eingangs genannten Art die Merkmale des Anspruchs 9 vorgesehen.

Alternativ sind zur Lösung der Aufgabe und insbesondere zur Durchführung des erfindungsgemäßen Verfahrens bei einem Gerät der eingangs genannten Art die Merkmale des Anspruchs 10 vorgesehen.

Insbesondere ist bei einem Gerät zur Bestimmung einer NOₓ-Konzentration in einem Messgas, welches einen NOₓ-sensitiven und mit dem Messgas in Kontakt bringbaren Gassensor und eine Auswerteeinheit zur Bestimmung eines Messwerts für die NOₓ-Konzentration im Messgas aus dem Sensorsignal des Gassensors hat, vorgesehen, dass ein weiterer Gassensor vorhanden ist, der zur Bestimmung der Konzentration eines zweiten Bestandteils im Messgas ausgebildet ist, und dass die Auswerteeinheit Mittel zur Bestimmung eines Zahlenwertes für die NOₓ-Konzentration im Messgas aus dem mit dem ersten Gassensor bestimmten Messwert für die NOₓ-Konzentration im Messgas und im Sensorsignal des zweiten Gassensors aufweist. Vorzugsweise sind an dem Gerät Mittel zur Durchführung des beschriebenen erfindungsgemäßen Verfahrens ausgebildet.

Bei einer vorteilhaften Ausgestaltung kann vorgesehen sein, dass das Gerät tragbar ausgebildet ist, beispielsweise indem die Komponenten des Geräts in einem gemeinsamen Gehäuse angeordnet sind.

Gemäß einer Ausgestaltung der Erfindung kann vorgesehen sein, dass in oder an dem Gerät ein Speichermittel vorgesehen ist, in welchem eine erste Kennlinie für die Auswertung des Sensorsignals des ersten Gassensors und eine zweite Kennlinie für die Bewertung des zweiten Sensorsignals hinterlegt sind, wobei aus der zweiten Kennlinie ein Differenzwert, um welchen der Zahlenwert von dem Messwert für die NOₓ-Konzentration im Messgas abweicht, oder ein Faktor, mit welchem der Messwert für die NOₓ-Konzentration zur Bestimmung eines korrigierten Messwerts, also des Zahlenwerts, zu multiplizieren ist, entnehmbar ist. Somit ist die Korrektur von systematischen Messfehlern aufgrund von den Einflüssen durch den zweiten Bestandteil, beziehungsweise die Simulation eines gesetzlich vorgeschriebenen oder bereits etablierten Messverfahrens besonders einfach durchführbar, und das erfindungsgemäße Gerät ist an sich ändernde gesetzliche Vorgaben oder die Auswahl eines anderen Referenzverfahrens einfach anpassbar. Die hinterlegten Kennlinien sind vorzugsweise durch Referenzmessungen gewonnen und als Datenreihe und/oder als funktionale Abhängigkeit hinterlegt.

In einer Weiterbildung sind für verschiedene Referenz-Messverfahren verschiedene Kennlinien hinterlegt, so dass für eine Mehrzahl von Messverfahren der jeweils sich ergebende Messwert für die NOₓ-Konzentration angebar ist. Zur Vereinfachung kann auch eine über verschiedene Messverfahren gemittelte Kennlinie bzw. deren Parameter hinterlegt sein.

Zur Erzielung einer verbesserten Berücksichtigung der durch weitere Bestandteile des Messgases hervorgerufenen Einflüsse kann vorgesehen sein, dass Mittel zur Eingabe und/oder Bestimmung der Konzentration eines weiteren Bestandteils im Messgas ausgebildet sind, und dass für verschiedene Zahlenwerte dieser Konzentration des weiteren Bestandteils verschiedene Kennlinien hinterlegt sind, aus denen jeweils der Differenzwert oder Faktor, um welchen der Zahlenwert von dem Messwert für die NOₓ-Konzentration im Messgas abweicht, entnehmbar ist. Dieser Differenzwert oder Faktor kann bei der Erfindung generell dadurch gegeben sein, dass der mit dem Messverfahren erzielte Messwert und der um systematische Messfehler korrigierte Zahlenwert hinterlegt sind, wobei die Differenz durch Differenzbildung entsteht, oder dass ein Faktor hinterlegt ist, mit welchem der Messwert zu multiplizieren ist, um zum korrigierten Messwert zu gelangen, wobei der Faktor durch Quotientenbildung entsteht, oder dass der Differenzwert oder der Faktor direkt hinterlegt ist. Für viele Anwendungsanforderungen ist es bereits ausreichend, wenn die Eingabe der Konzentration eines weiteren Bestandteils im Messgas in diskreten Stufungen ermöglicht ist. Beispielsweise kann der weitere Bestandteil der Wassergehalt im Messgas und also der Feuchtigkeitsgehalt des Messgases sein.

Bei einer Ausgestaltung der Erfindung kann vorgesehen sein, dass ein Gaskühler und/oder eine Kondensatfalle im Gasstrom des Messgases vor dem Gassensor oder den Gassensoren angeordnet ist. Von Vorteil ist dabei, dass durch den vorgeschalteten Gaskühler beziehungsweise die vorgeschaltete Kondensatfalle ein definierter Feuchtigkeitsgehalt des Messgases eingestellt wird, wodurch systematische Messfehler reduziert sind.

Zur Berücksichtigung des Einflusses des Feuchtigkeitsgehaltes im Messgas kann vorgesehen sein, dass Mittel zur Erkennung des Vorhandenseins eines Gaskühlers oder einer Kondensatfalle im Gasstrom des Messgases und/oder Mittel zur Erkennung und/oder Eingabe der Arbeitstemperatur des Gaskühlers oder der Kondensatfalle vorgesehen sind. Durch diese Mittel ist bereits ein Rückschluss auf den für die Korrektur von systematischen Messfehlern verwendbaren Feuchtigkeitsgehalt im Messgas zumindest in groben Stufungen möglich.

Zur Bestimmung des Feuchtigkeitsgehalts im Messgas kann vor dem Gaskühler oder allgemein einer Gastrocknungseinrichtung oder statt der Gastrocknungseinrichtung ein Feuchtigkeitssensor, beispielsweise eine ZrO₂-Sonde, mit dem Messgas in Kontakt gebracht sein. Mit den Messwerten für die Feuchtigkeit sind die korrigierten Messwerte für die NOₓ-Konzentration in trockenem Messgas auf feuchtes Messgas umrechenbar und umgekehrt.

Wird das erfindungsgemäße Gerät zur Bestimmung des NOₓ-Anteils im Abgas eines Verbrennungsmotors eingesetzt, so können zur Bestimmung des Feuchtigkeitsgehalts Sensoren und/oder Eingabemittel vorgesehen sein, mit denen Parameter des eingesetzten Brennstoffs, beispielsweise der H- oder C-Anteil, und der zugeführten Luft, beispielsweise der O₂-Anteil, ermittelbar bzw. eingebbar sind. In diesem Fall sind an dem Gerät Mittel ausgebildet, die eine Berechnung des Feuchtigkeitsgehalts im Abgas aus diesen Parametern erlauben.

Die Erfindung wird nun anhand von Ausführungsbeispielen beschrieben, ist aber nicht auf diese Ausführungsbeispiele beschränkt. Weitere Ausführungsbeispiele ergeben sich durch Kombination der Merkmale der Patentansprüche untereinander und/oder mit Merkmalen der Ausführungsbeispiele.

Es zeigt
- Fig. 1:: eine erfindungsgemäße Anordnung zur Bestimmung der NOₓ-Konzentration in einem Motorabgas und
- Fig. 2:: den Einfluss der Sauerstoffkonzentration auf die mit unterschiedlichen Messverfahren bestimmte NOₓ-Konzentration.

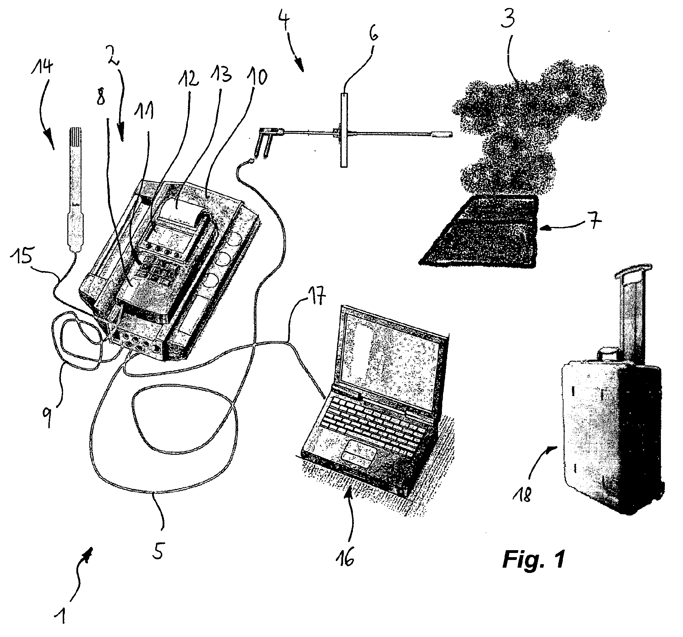

Figur 1 zeigt einen skizzenhaften Prinzipaufbau einer allgemein mit 1 bezeichneten Anordnung mit einem Gerät 2 zur Bestimmung einer NOₓ-Konzentration in einem Messgas 3.

Die Anordnung hat hierzu eine Sonde 4, mit der ein Anteil des Messgases 3 entnommen und über einen unbeheizten Schlauch 5 dem Gerät 2 zugeführt wird. Die Zuführung des Messgases 3 über den Schlauch 5 erfolgt wie in DE 196 31 002 C2, insbesondere in Seite 3, Zeile 29 bis Seite 4, Zeile 10 und in den Patentansprüchen dieser Druckschrift beschrieben.

Bei einem weiteren Ausführungsbeispiel ist der Schlauch 5 beheizbar.

Zur Entnahme der Messgasprobe in Fig. 1 wird die Sonde 4 mit ihrem Befestigungsflansch 6 an dem Schornstein 7 befestigt, aus welchem das Messgas 3 entnommen wird.

Das Gerät 2 ist je nach Anforderung zur Bestimmung der Konzentration von O₂, CO, NO, NO₂ und SO₂ mit Hilfe von elektrochemischen Sensoren ausgerüstet und verfügt zusätzlich über einen CO₂-IR-Sensor inklusive absoluter Druckmessung.

Das Gerät 2 verfügt über eine Auswerteeinheit 8 mit Mitteln zur Bestimmung eines Zahlenwertes für die NOₓ-Konzentration im Messgas aus den mit dem ersten Gassensor bestimmten Messwert für die NOₓ-Konzentration im Messgas 3 und dem Sensorsignal des zweiten Gassensors für die O₂-Konzentration im Messgas 3.

Die Auswerteeinheit 8 ist über eine Datenleitung 9 an den Grundkörper 10 des Gerätes 2 angeschlossen, der die Sensoren zur Bestimmung der Konzentrationen der Bestandteile in dem über die Sonde 4 entnommenen Anteil des Messgases 3 gehäusebildend umschließt.

An der Auswerteeinheit 8, die von dem Grundkörper 10 trennbar ist, sind Eingabemittel 11 zur Eingabe von Bedienbefehlen und/oder Zahlenwerten, Anzeigemittel 12 zur Anzeige von Bedienschritten, Eingabeaufforderungen beziehungsweise Messwerten und/oder berechneten Zahlenwerten und Ausgabemittel 13 zur Ausgabe von Messprotokollen und Messwerten vorgesehen.

In der Auswerteeinheit 8 ist ein Speichermittel angeordnet, in welchem eine erste Kennlinie für die Auswertung des Sensorsignals des NOₓ-Gassensors und eine zweite Kennlinie für die Bewertung des zweiten Sensorsignals des O₂-Sensors hinterlegt sind.

An die Auswerteeinheit 8 ist zur Bestimmung von Umgebungsfeuchtigkeit und Umgebungstemperatur ein weiteres Messgerät 14 über eine weitere Datenleitung 15 angeschlossen.

Zur Weiterverarbeitung der ermittelten Messwerte und/oder des Messprotokolls ist ein PC 16 über eine Verbindungsleitung 17 an die Auswerteeinheit 8 angeschlossen. Die Komponenten der Anordnung 1 sind in einem Zubehörkoffer 18 zum Transport verstaubar.

Bei dem in Figur 1 dargestellten Gerät 2 ist zur Bestimmung des NOₓ-Bestandteils im Messgas 3 ein elektrochemischer Sensor vorgesehen, und es ist in der Auswerteeinheit 8 eine Kennlinie hinterlegt, mit welcher das Sensorsignal des O₂-Sensors bewertbar ist zur Simulation der Messung der NOₓ-Konzentration mit einem CLD-Sensor.

Bei einem weiteren Ausführungsbeispiel kann vorgesehen sein, dass die Auswerteeinheit 8 in Figur 1 einen CLD-Sensor zur Bestimmung der NOₓ-Konzentration enthält und dass in der Auswerteeinheit 8 eine Kennlinie für die Bewertung des Sensorsignals des O₂-Sensors hinterlegt ist, mit welcher die beschriebene Messwertabweichung des CLD-Sensors bei hohem O₂-Anteil im Messgas korrigierbar ist. Bei diesem Ausführungsbeispiel ist der Schlauch 5 zur Zuführung des Messgases beheizt, und es ist bei Messung am trockenen Abgas im Messgasstrom ein Gaskühler, bei Messung am feuchten Abgas im Messgasstrom kein Gaskühler vorgesehen.

Mit der beschriebenen Anordnung ist ein Verfahren zur Bestimmung einer NOₓ-Konzentration in dem Messgas 3 ausführbar, das im Folgenden näher beschrieben wird.

In der Auswerteeinheit 8 wird ein Sensorsignal eines in dem Grundkörper 10 angeordneten, für NOₓ-sensitiven Gassensors erfasst, welcher über die Sonde 4 und den Schlauch 5 mit dem Messgas 3 in Kontakt gebracht ist.

Aus diesem erfassten Sensorsignal wird sodann ein Messwert für die NOₓ-Konzentration bestimmt.

Mittels eines weiteren Sensors im Grundkörper 10 des Gerätes 2 wird ein Messwert für die Konzentration von O₂ im Messgas 3 ermittelt.

Aus den beiden ermittelten Messwerten für die Konzentration von NOₓ und O₂ wird nun ein Zahlenwert für die NOₓ-Konzentration im Messgas 3 bestimmt, der sich bei Verwendung eines CLD-Sensors statt des elektrochemischen Sensors ergeben würde.

Der mit dem elektrochemischen Sensor gemessene Messwert für die NOₓ-Konzentration im Messgas 3 und der bestimmte Zahlenwert, der sich bei Verwendung eines CLD-Sensors ergeben würde, werden an dem Anzeigemittel 12 der Auswerteeinheit 8 angezeigt.

Zur Bestimmung der Messwerte und Zahlenwerte sind in der Anzeigeeinheit 8 Kennlinien hinterlegt.

Das Gerät 2 verfügt im Grundkörper 10 weiter über einen in der Abbildung nicht ersichtlichen Gaskühler, der im Messgasstrom den Gassensoren vorgeschaltet ist. Somit messen die Gassensoren im Grundkörper 10 den trockenen Messgasstrom. Mit der Kenntnis des Feuchtigkeitsgehalts des Messgases 3 vor Eintritt in den Gaskühler ist hieraus die NOₓ-Konzentration für feuchtes Messgas berechenbar.

Bei einem weiteren Ausführungsbeispiel ist der Gaskühler separat ausgebildet.

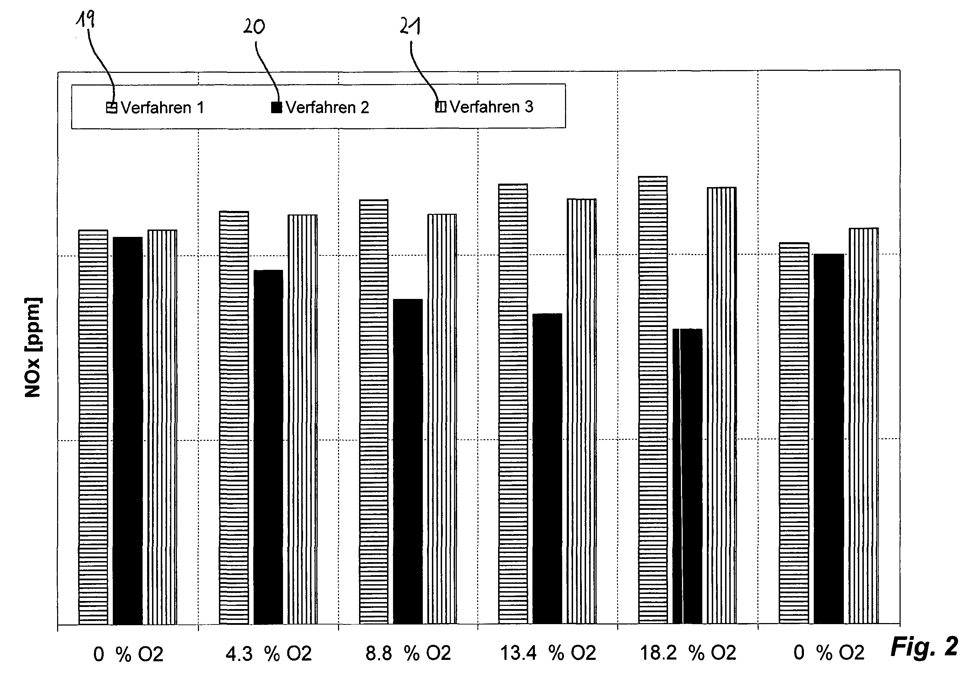

Figur 2 zeigt das Ergebnis einer Messreihe, bei der die NOₓ-Konzentration in einem Messgas bei unterschiedlichen Sauerstoffkonzentrationen mit unterschiedlichen Messverfahren bestimmt wurde. Dargestellt sind die ermittelten NOₓ-Konzentrationen mit einem nicht dispersiven Infrarot-Sensor 19, mit einem Chemilumineszenz-Detektor 20 und mit einem elektrochemischen Sensor 21.

Die Abszisse stellt dabei die mit den unterschiedlichen Verfahren gemessenen Werte für die NOₓ-Konzentration bei verschiedenen Sauerstoff-Konzentrationen im Messgas dar. Deutlich sind Abweichungen mit zunehmendem Sauerstoffgehalt ersichtlich.

Deutlich ist erkennbar, dass die mit dem nicht dispersiven Infrarot-Sensor 19 erzielten Messwerte mit den mittels des elektrochemischen Sensors 21 erzielten Messwerte im Rahmen von statistischen Schwankungen übereinstimmen, während sich zu den Messwerten 20 des CLD-Sensors Abweichungen ergeben, die betragsmäßig mit zunehmendem Sauerstoff-Gehalt zunehmen.

Aus diesen Informationen ist eine Kennlinie ableitbar, mit der die aus einem CLD-Sensor ermittelte NOₓ-Konzentration bei Kenntnis der O₂-Konzentration korrigierbar ist, beziehungsweise mit der die Messung mit einem CLD-Sensor auf der Grundlage einer Messung der NOₓ-Konzentration mit einem IR- beziehungsweise elektrochemischen Sensor simulierbar ist.

Die erwähnte Information ist in Form von Kennlinien in der Auswerteeinheit 8 hinterlegt und dient zur Bestimmung des Zahlenwerts für die NOₓ-Konzentration, welcher an dem Anzeigemittel 12 angezeigt und gegebenenfalls mit dem Ausgabemittel 13 ausgegeben wird.

In der Auswerteeinheit 8 sind gesetzlich oder als Referenz vorgegebene Grenzwerte für NOₓ-Konzentrationen abgespeichert, und es wird die Über- und/oder Unterschreitung von diesen Grenzwerten jeweils für den Messwert und den Zahlenwert für die NOₓ-Konzentration im Messgas getrennt am Anzeigemittel 12 signalisiert beziehungsweise in einem Messprotokoll protokolliert. In der Auswerteeinheit 8 sind für weitere Bestandteile, insbesondere CO₂ und Wasser, im Messgas 3 analog zu Figur 2 die Abweichung der verschiedenen Messverfahren voneinander hinterlegt, so dass die von den weiteren Bestandteilen hervorgerufenen Einflüsse auf die Bestimmung der NOₓ-Konzentration berücksichtigbar sind.

In der Auswerteeinheit 8 werden die Sensorsignale von Gassensoren in dem Grundkörper 10 schließlich derart verarbeitet, dass eine Berechnung des Feuchtigkeitsgehalts des Messgases 3 vor Eintritt in den Gaskühler möglich ist.

Bei dem Verfahren zur Bestimmung einer NOₓ-Konzentration in einem Messgas ist vorgesehen, dass ein Messwert für die NOₓ-Konzentration aus dem Sensorsignal eines Gassensors bestimmt wird und dass ein Messwert für die Konzentration eines zweiten Bestandteils im Messgas ermittelt wird, wobei aus den Messwerten ein korrigierter Wert für die NOₓ-Konzentration im Messgas bestimmt wird und wobei der Messwert und der korrigierte Messwert für die NOₓ-Konzentration angezeigt und/oder ausgegeben werden.

## Patentansprüche

1. Verfahren zur Bestimmung einer NOₓ-Konzentration in einem Messgas, wobei ein Sensorsignal eines für NOₓ sensitiven und mit dem Messgas in Kontakt gebrachten Gassensors erfasst wird und aus dem Sensorsignal ein Messwert für die NOₓ-Konzentration bestimmt wird, wobei ein Messwert für die Konzentration eines zweiten Bestandteils im Messgas mit einem zweiten Gassensor ermittelt wird, wobei der zweite Bestandteil Sauerstoff ist, und wobei aus dem Messwert für die Konzentration des zweiten Bestandteils im Messgas ein Zahlenwert für die NOₓ-Konzentration im Messgas bestimmt wird, wobei der Messwert für die NOₓ-Konzentration in Messgas anhand einer hinterlegten ersten Kennlinie (19, 20, 21) aus dem Sensorsignal des ersten Gassensors bestimmt wird und der Zahlenwert für die NOₓ-Konzentration in Messgas anhand einer hinterlegten zweiten Kennlinie aus dem Sensorsignal des ersten Gassensors und des zweiten Gassensors bestimmt wird, wobei die erste Kennlinie (19, 20, 21) die Kennlinie eines NOₓ-sensitiven Infrarot-Sensors, UV-Sensors, elektrochemischen Sensors oder Halbleitersensors ist und die zweite Kennlinie die Abweichungen der Kennlinie eines NOₓ-sensitiven Chemilumineszenz-Detektors von der ersten Kennlinie bei unterschiedlichen Werten der Konzentration des weiteren Bestandteils im Messgas abbildet.

2. Verfahren zur Bestimmung einer NOₓ-Konzentration in einem Messgas, wobei ein Sensorsignal eines für NOₓ sensitiven und mit dem Messgas in Kontakt gebrachten Gassensors erfasst wird und aus dem Sensorsignal ein Messwert für die NOₓ-Konzentration bestimmt wird, wobei ein Messwert für die Konzentration eines zweiten Bestandteils im Messgas mit einem zweiten Gassensor ermittelt wird, wobei der zweite Bestandteil Sauerstoff ist, und wobei aus dem Messwert für die Konzentration des zweiten Bestandteils im Messgas ein Zahlenwert für die NOₓ-Konzentration im Messgas bestimmt wird, wobei der Messwert für die NOₓ-Konzentration im Messgas anhand einer hinterlegten ersten Kennlinie (19, 20, 21) aus dem Sensorsignal des ersten Gassensors bestimmt wird und der Zahlenwert für die NOₓ-Konzentration im Messgas anhand einer hinterlegten zweiten Kennlinie aus dem Sensorsignal des ersten Gassensors und des zweiten Gassensors bestimmt wird, wobei die erste Kennlinie (19, 20, 21) die Kennlinie eines Chemilumineszenz-Detektors ist und die zweite Kennlinie die Abweichungen der Kennlinie eines NOₓ-sensitiven elektrochemischen Sensors, Infrarot-Sensors, UV-Sensors oder Halbleitersensors von der ersten Kennlinie bei unterschiedlichen Werten der Konzentration des weiteren Bestandteils im Messgas abbildet.

3. Verfahren nach Anspruch 1 oder 2, **dadurch gekennzeichnet, dass** der Messwert und der Zahlenwert für die NOₓ-Konzentration im Messgas angezeigt werden.

4. Verfahren nach einem der Ansprüche 1 bis 3, **dadurch gekennzeichnet, dass** das Messgas aus den Abgasen eines Verbrennungsmotors entnommen wird.

5. Verfahren nach einem der Ansprüche 1 bis 4, **dadurch gekennzeichnet, dass** die erste Kennlinie (19, 20, 21) und/oder die zweite Kennlinie durch Interpolation oder Extrapolation von Kalibrier- oder Stützpunkten jeweils einer Kennlinienkurve gebildet ist/sind.

6. Verfahren nach einem der Ansprüche 1 bis 5, **dadurch gekennzeichnet, dass** die Über- und/oder Unterschreitung von Grenzwerten für Messwert und Zahlenwert für die NOₓ-Konzentration im Messgas getrennt signalisiert wird.

7. Verfahren nach einem der Ansprüche 1 bis 6, **dadurch gekennzeichnet, dass** wenigstens ein weiterer Mess- und/oder Kennwert für die Konzentration eines weiteren Bestandteils im Messgas ermittelt und/oder eingegeben wird und dass aus dem weiteren Mess- und/oder Kennwert ein Korrekturwert für die NOₓ-Konzentration im Messgas bestimmt wird und/oder dass die Bestimmung des Zahlenwertes für die NOₓ-Konzentration im Messgas aus dem Messwert für die Konzentration des zweiten Bestandteils und/oder des weiteren Bestandteils in Abhängigkeit vom Feuchtigkeitsgehalt des Messgases erfolgt.

8. Verfahren nach einem der Ansprüche 1 bis 7, **dadurch gekennzeichnet, dass** das Messgas ein Abgas eines Verbrennungsmotors ist und dass bei den dem Verbrennungsmotor zugeführten chemischen Substanzen, insbesondere dem Kraftstoff, über eine Elementaranalyse die Konzentration von Elementarbestandteilen, insbesondere H und/oder C, und/oder der Feuchtigkeitsgehalt der dem Verbrennungsmotor zugeführten Luft bestimmt werden und/oder dass der Feuchtigkeitsgehalt des Messgases berechnet wird, insbesondere aus den bestimmten Konzentrationen der Elementarbestandteile und/oder dem Feuchtigkeitsgehalt der dem Verbrennungsmotor zugeführten Luft und/oder dass der Feuchtigkeitsgehalt im Messgas erfasst wird.

9. Gerät zur Bestimmung einer NOₓ-Konzentration in einem Messgas, mit einem für NOₓ-sensitiven und mit dem Messgas in Kontakt bringbaren Gassensor und einer Auswerteeinheit (8) zur Bestimmung eines Messwerts für die NOₓ-Konzentration im Messgas (3) aus dem Sensorsignal des Gassensors, **dadurch gekennzeichnet, dass** ein O₂-Sensor als ein weiterer Gassensor vorgesehen ist, der zur Bestimmung der Konzentration von Sauerstoff als ein zweiter Bestandteil im Messgas (3) ausgebildet ist, und dass die Auswerteeinheit (8) Mittel zur Bestimmung eines Zahlenwertes für die NOₓ-Konzentration im Messgas (3) aus dem mit dem ersten Gassensor bestimmten Messwert für die NOₓ-Konzentration im Messgas und dem Sensorsignal des zweiten Gassensors aufweist und dass ein Speichermittel vorgesehen ist, in welchem eine erste Kennlinie (19, 20, 21) für die Auswertung des Sensorsignals des ersten Gassensors und eine zweite Kennlinie für die Bewertung des zweiten Sensorssignals hinterlegt sind, wobei die erste Kennlinie (19, 20, 21) die Kennlinie eines Infrarot-Sensors, UV-Sensors, elektrochemischen Sensors oder Halbleitersensors ist und die zweite Kennlinie die Abweichungen der Kennlinie eines Chemilumineszenz-Detektors von der ersten Kennlinie bei unterschiedlichen Werten der Konzentration des zweiten Bestandteils abbildet.

10. Gerät zur Bestimmung einer NOₓ-Konzentration in einem Messgas, mit einem für NOₓ-sensitiven und mit dem Messgas in Kontakt bringbaren Gassensor und einer Auswerteeinheit (8) zur Bestimmung eines Messwerts für die NOₓ-Konzentration im Messgas (3) aus dem Sensorsignal des Gassensors, **dadurch gekennzeichnet, dass** ein O₂-Sensor als ein weiterer Gassensor vorgesehen ist, der zur Bestimmung der Konzentration von Sauerstoff als ein zweiter Bestandteil im Messgas (3) ausgebildet ist, und dass die Auswerteeinheit (8) Mittel zur Bestimmung eines Zahlenwertes für die NOₓ-Konzentration im Messgas (3) aus dem mit dem ersten Gassensor bestimmten Messwert für die NOₓ-Konzentration im Messgas und dem Sensorsignal des zweiten Gassensors aufweist und dass ein Speichermittel vorgesehen ist, in welchem eine erste Kennlinie für die Auswertung der Sensorsignale des ersten Gassensors und eine zweite Kennlinie für die Bewertung des zweiten Sensorssignals hinterlegt sind, wobei die erste Kennlinie (19, 20, 21) die Kennlinie eines Chemilumineszenz-Detektors ist und die zweite Kennlinie die Abweichungen der Kennlinie eines NOₓ-sensitiven elektrochemischen Sensors, Infrarot-Sensors, UV-Sensors oder Halbleitersensors von der ersten Kennlinie bei unterschiedlichen Werten der Konzentration des zweiten Bestandteils im Messgas abbildet.

11. Gerät nach Anspruch 9 oder 10, **dadurch gekennzeichnet, dass** Mittel zur Eingabe (11) und/oder Bestimmung der Konzentration eines weiteren Bestandteils im Messgas ausgebildet sind, und dass für verschiedene Zahlenwerte dieser Konzentration des weiteren Bestandteils verschiedene Kennlinien (19, 20, 21) hinterlegt sind, aus denen jeweils der Differenzwert oder der Faktor entnehmbar ist, um welchen der Zahlenwert von dem Messwert für die NOₓ-Konzentration im Messgas abweicht.

12. Gerät nach einem der Ansprüche 9 bis 11, **dadurch gekennzeichnet, dass** ein Gaskühler oder eine Kondensatfalle im Gasstrom des Messgases angeordnet ist und/oder dass Mittel zur Erkennung des Vorhandenseins eines Gaskühlers oder einer Kondensatfalle im Gasstrom des Messgases und/oder Mittel zur Erkennung und/oder Eingabe der Arbeitstemperatur des Gaskühlers oder der Kondensatfalle und/oder Mittel zur Bestimmung des Feuchtigkeitsgehalts im Messgas, insbesondere vor Eintritt in einen Gaskühler, vorgesehen sind.

13. Gerät nach einem der Ansprüche 9 bis 12, **dadurch gekennzeichnet, dass** der zweite Gassensor ein elektrochemischer O₂-Sensor ist und/oder dass das Gerät (2) tragbar ausgebildet ist.

## Claims

1. Method for determining an NOₓ concentration in a measurement gas, wherein a sensor signal from a gas sensor which is sensitive to NOₓ and is brought into contact with the measurement gas is detected and from the sensor signal, a measurement value for the NOₓ concentration is determined, wherein a measurement value for the concentration of a second component in the measurement gas is determined using a second gas sensor, wherein the second component is oxygen, and wherein a numerical value for the NOₓ concentration in the measurement gas is determined from the measurement value for the concentration of the second component in the measurement gas, wherein the measurement value for the NOₓ concentration in the measurement gas is determined with reference to a stored first characteristic line (19, 20, 21) from the sensor signal of the first gas sensor and the numerical value for the NOₓ concentration in the measurement gas is determined with reference to a stored second characteristic line from the sensor signal of the first gas sensor and of the second gas sensor, wherein the first characteristic line (19, 20, 21) is the characteristic line of an NOₓ-sensitive infrared sensor, UV sensor, electrochemical sensor or semiconductor sensor and the second characteristic line maps the deviations of the characteristic line of an NOₓ-sensitive chemiluminescence detector from the first characteristic line in the case of different values of the concentration of the further component in the measurement gas.

2. Method for determining an NOₓ concentration in a measurement gas, wherein a sensor signal from a gas sensor which is sensitive to NOₓ and is brought into contact with the measurement gas is detected and from the sensor signal, a measurement value for the NOₓ concentration is determined, wherein a measurement value for the concentration of a second component in the measurement gas is determined using a second gas sensor, wherein the second component is oxygen, and wherein a numerical value for the NOₓ concentration in the measurement gas is determined from the measurement value for the concentration of the second component in the measurement gas, wherein the measurement value for the NOₓ concentration in the measurement gas is determined with reference to a stored first characteristic line (19, 20, 21) from the sensor signal of the first gas sensor and the numerical value for the NOₓ concentration in the measurement gas is determined with reference to a stored second characteristic line from the sensor signal of the first gas sensor and of the second gas sensor, wherein the first characteristic line (19, 20, 21) is the characteristic line of a chemiluminescence detector and the second characteristic line maps the deviations of the characteristic line of an NOₓ-sensitive electrochemical sensor, infrared sensor, UV sensor or semiconductor sensor from the first characteristic line in the case of different values of the concentration of the further component in the measurement gas.

3. Method as claimed in claim 1 or 2, **characterised in that** the measurement value and the numerical value for the NOₓ concentration in the measurement gas are displayed.

4. Method as claimed in any one of claims 1 to 3, **characterised in that** the measurement gas is taken from the exhaust gases of an internal combustion engine.

5. Method as claimed in any one of claims 1 to 4, **characterised in that** the first characteristic line (19, 20, 21) and/or the second characteristic line is/are formed by interpolation or extrapolation of calibration or support points of a respective characteristic line curve.

6. Method as claimed in any one of claims 1 to 5, **characterised in that** exceeding and/or falling short of limit values for the measurement value and numerical value for the NOₓ concentration in the measurement gas is separately signalled.

7. Method as claimed in any one of claims 1 to 6, **characterised in that** at least one further measurement and/or characteristic value for the concentration of a further component in the measurement gas is determined and/or input and that a correction value for the NOₓ concentration in the measurement gas is determined from the further measurement and/or characteristic value and/or that the numerical value for the NOₓ concentration in the measurement gas is determined from the measurement value for the concentration of the second component and/or the further component in dependence upon the moisture content of the measurement gas.

8. Method as claimed in any one of claims 1 to 7, **characterised in that** the measurement gas is an exhaust gas of an internal combustion engine and that in the case of the chemical substances supplied to the internal combustion engine, in particular the fuel, an elementary analysis is used to determine the concentration of elementary components, in particular H and/or C, and/or the moisture content of the air fed to the internal combustion engine and/or that the moisture content of the measurement gas is calculated, in particular from the determined concentrations of the elementary components and/or the moisture content of the air fed to the internal combustion engine and/or that the moisture content in the measurement gas is detected.

9. Device for determining an NOₓ concentration in a measurement gas, having a gas sensor which is sensitive to NOₓ and can be brought into contact with the measurement gas, and having an evaluation unit (8) for determining a measurement value for the NOₓ concentration in the measurement gas (3) from the sensor signal of the gas sensor, **characterised in that** an O₂ sensor is provided as a further gas sensor and is designed to determine the concentration of oxygen as a second component in the measurement gas (3), and that the evaluation unit (8) has means for determining a numerical value for the NOₓ concentration in the measurement gas (3) from the measurement value, determined using the first gas sensor, for the NOₓ concentration in the measurement gas, and from the sensor signal of the second gas sensor, and that a storage means is provided in which a first characteristic line (19, 20, 21) for evaluation of the sensor signal of the first gas sensor and a second characteristic line for evaluation of the second sensor signal are stored, wherein the first characteristic line (19, 20, 21) is the characteristic line of an infrared sensor, UV sensor, electrochemical sensor or semiconductor sensor and the second characteristic line maps the deviations of the characteristic line of a chemiluminescence detector from the first characteristic line in the case of different values for the concentration of the second component.

10. Device for determining an NOₓ concentration in a measurement gas, having a gas sensor which is sensitive to NOₓ and can be brought into contact with the measurement gas, and having an evaluation unit (8) for determining a measurement value for the NOₓ concentration in the measurement gas (3) from the sensor signal of the gas sensor, **characterised in that** an O₂ sensor is provided as a further gas sensor and is used to determine the concentration of oxygen as a second component in the measurement gas (3), and that the evaluation unit (8) has means for determining a numerical value for the NOₓ concentration in the measurement gas (3) from the measurement value, determined using the first gas sensor, for the NOx concentration in the measurement gas and from the sensor signal of the second gas sensor, and that a storage means is provided in which a first characteristic line for evaluation of the sensor signals of the first gas sensor and a second characteristic line for evaluation of the second sensor signal are stored, wherein the first characteristic line (19, 20, 21) is the characteristic line of a chemiluminescence detector and the second characteristic line maps the deviations of the characteristic line of an NOx-sensitive electrochemical sensor, infrared sensor, UV sensor, or semiconductor sensor from the first characteristic line in the case of different values for the concentration of the second component in the measurement gas.

11. Device as claimed in claim 9 or 10, **characterised in that** means are formed for inputting (11) and/or determining the concentration of a further component in the measurement gas, and that for different numerical values of this concentration of the further component, different characteristic lines (19, 20, 21) are stored, from which in each case it is possible to derive the difference value or factor by which the numerical value deviates from the measurement value for the NOₓ concentration in the measurement gas.

12. Device as claimed in any one of claims 9 to 11, **characterised in that** a gas cooler or condensate trap is disposed in the gas flow of the measurement gas and/or that means for identifying the presence of a gas cooler or of a condensate trap in the gas flow of the measurement gas and/or means for detecting and/or inputting the working temperature of the gas cooler or of the condensate trap and/or means for determining the moisture content in the measurement gas, in particular prior to entry into a gas cooler, are provided.

13. Device as claimed in any one of claims 9 to 12, **characterised in that** the second gas sensor is an electrochemical O₂ sensor and/or that the device (2) is of portable construction.

## Revendications

1. Procédé pour la détermination d'une concentration en NOₓ dans un gaz de mesure, dans lequel on détecte un signal de détecteur d'un détecteur de gaz sensible aux NOₓ et mis en contact avec un gaz de mesure et on déduit du signal de détecteur une valeur de mesure de la concentration en NOₓ, dans lequel on détermine une valeur de mesure pour la concentration d'un deuxième composant dans le gaz de mesure avec un deuxième détecteur de gaz, dans lequel le deuxième composant est l'oxygène, et dans lequel on détermine à partir de la valeur de mesure pour la concentration du deuxième composant dans le gaz de mesure une valeur chiffrée pour la concentration en NOₓ dans le gaz de mesure, dans lequel on détermine la valeur de mesure pour la concentration en NOₓ dans le gaz de mesure à l'aide d'une première courbe caractéristique mémorisée (19, 20, 21) à partir du signal de détecteur du premier détecteur de gaz et on détermine la valeur chiffrée pour la concentration en NOₓ dans le gaz de mesure à l'aide d'une deuxième courbe caractéristique mémorisée à partir du signal de détecteur du premier détecteur de gaz et du deuxième détecteur de gaz, dans lequel la première courbe caractéristique (19, 20, 21) est la courbe caractéristique d'un détecteur infrarouge, d'un détecteur UV, d'un détecteur électrochimique ou d'un détecteur à semi-conducteur sensible aux NOₓ et la deuxième courbe caractéristique représente les écarts de la courbe caractéristique d'un détecteur par chimioluminescence sensible aux NOₓ par rapport à la première courbe caractéristique pour différentes valeurs de la concentration de l'autre composant dans le gaz de mesure.

2. Procédé pour la détermination d'une concentration en NOₓ dans un gaz de mesure, dans lequel on détecte un signal de détecteur d'un détecteur de gaz sensible aux NOₓ et mis en contact avec un gaz de mesure et on déduit du signal de détecteur une valeur de mesure de la concentration en NOₓ, dans lequel on détermine une valeur de mesure pour la concentration d'un deuxième composant dans le gaz de mesure avec un deuxième détecteur de gaz, dans lequel le deuxième composant est l'oxygène, et dans lequel on détermine à partir de la valeur de mesure pour la concentration du deuxième composant dans le gaz de mesure une valeur chiffrée pour la concentration en NOₓ dans le gaz de mesure, dans lequel on détermine la valeur de mesure pour la concentration en NOₓ dans le gaz de mesure à l'aide d'une première courbe caractéristique mémorisée (19, 20, 21) à partir du signal de détecteur du premier détecteur de gaz et on détermine la valeur chiffrée pour la concentration en NOₓ dans le gaz de mesure à l'aide d'une deuxième courbe caractéristique mémorisée à partir du signal de détecteur du premier détecteur de gaz et du deuxième détecteur de gaz, dans lequel la première courbe caractéristique (19, 20, 21) est la courbe caractéristique d'un détecteur par chimioluminescence et la deuxième courbe caractéristique représente les écarts de la courbe caractéristique d'un détecteur électrochimique, d'un détecteur infrarouge, d'un détecteur UV ou d'un détecteur à semi-conducteur sensible aux NOₓ par rapport à la première courbe caractéristique pour différentes valeurs de la concentration de l'autre composant dans le gaz de mesure.

3. Procédé selon la revendication 1 ou 2, **caractérisé en ce que** l'on affiche la valeur de mesure et la valeur chiffrée pour la concentration en NOₓ dans le gaz de mesure.

4. Procédé selon l'une quelconque des revendications 1 à 3, **caractérisé en ce que** l'on prélève le gaz de mesure dans les gaz d'échappement d'un moteur à combustion interne.

5. Procédé selon l'une quelconque des revendications 1 à 4, **caractérisé en ce que** la première courbe caractéristique (19, 20, 21) et/ou la deuxième courbe caractéristique est/sont formée(s) par interpolation ou par extrapolation de points de calibrage ou de reprise respectivement d'une courbe caractéristique.

6. Procédé selon l'une quelconque des revendications 1 à 5, **caractérisé en ce que** l'on signale séparément le dépassement à la hausse et/ou à la baisse de valeurs limites pour la valeur de mesure et la valeur chiffrée pour la concentration en NOₓ dans le gaz de mesure.

7. Procédé selon l'une quelconque des revendications 1 à 6, **caractérisé en ce que** l'on détermine et/ou on introduit au moins une autre valeur de mesure et/ou une autre valeur caractéristique pour la concentration d'un autre composant dans le gaz de mesure et **en ce que** l'on détermine à partir de l'autre valeur de mesure et/ou caractéristique une valeur de correction pour la concentration en NOₓ dans le gaz de mesure et/ou **en ce que** l'on effectue la détermination de la valeur chiffrée pour la concentration en NOₓ dans le gaz de mesure à partir de la valeur de mesure pour la concentration du deuxième composant et/ou de l'autre composant en fonction de la teneur en humidité du gaz de mesure.

8. Procédé selon l'une quelconque des revendications 1 à 7, **caractérisé en ce que** le gaz de mesure est un gaz d'échappement d'un moteur à combustion interne et **en ce que**, parmi les substances chimiques fournies au moteur à combustion interne, en particulier le carburant, on détermine par une analyse élémentaire la concentration de composants élémentaires, en particulier H et/ou C, et/ou la teneur en humidité de l'air fourni au moteur à combustion interne, et/ou **en ce que** l'on calcule la teneur en humidité du gaz de mesure, en particulier à partir des concentrations déterminées des composants élémentaires et/ou de la teneur en humidité de l'air fourni au moteur à combustion interne et/ou **en ce que** l'on détecte la teneur en humidité dans le gaz de mesure.

9. Dispositif pour la détermination d'une concentration en NOₓ dans un gaz de mesure, avec un détecteur de gaz sensible aux NOₓ et pouvant être mis en contact avec le gaz de mesure et une unité d'exploitation (8) pour la détermination d'une valeur de mesure pour la concentration en NOₓ dans le gaz de mesure (3) à partir du signal de détecteur du détecteur de gaz, **caractérisé en ce qu'**il est prévu un détecteur d'O₂ comme autre détecteur de gaz, qui est configuré pour la détermination de la concentration en oxygène comme deuxième composant dans le gaz de mesure (3), et **en ce que** l'unité d'exploitation (8) présente des moyens pour la détermination d'une valeur chiffrée pour la concentration en NOₓ dans le gaz de mesure (3) à partir de la valeur de mesure déterminée avec le premier détecteur de gaz pour la concentration en NOₓ dans le gaz de mesure et du signal de détecteur du deuxième détecteur de gaz, et **en ce qu'**il est prévu un moyen de mémorisation, dans lequel une première courbe caractéristique (19, 20, 21) pour l'exploitation du signal de détecteur du premier détecteur de gaz et une deuxième courbe caractéristique pour l'évaluation du deuxième signal de détecteur sont mémorisées, dans lequel la première courbe caractéristique (19, 20, 21) est la courbe caractéristique d'un détecteur infrarouge, d'un détecteur UV, d'un détecteur électrochimique ou d'un détecteur à semi-conducteur et la deuxième caractéristique représente les écarts de la courbe caractéristique d'un détecteur par chimioluminescence par rapport à la première courbe caractéristique pour différentes valeurs de la concentration du deuxième composant.

10. Dispositif pour la détermination d'une concentration en NOₓ dans un gaz de mesure, avec un détecteur de gaz sensible aux NOₓ et pouvant être mis en contact avec le gaz de mesure et une unité d'exploitation (8) pour la détermination d'une valeur de mesure pour la concentration en NOₓ dans le gaz de mesure (3) à partir du signal de détecteur du détecteur de gaz, **caractérisé en ce qu'**il est prévu un détecteur d'O₂ comme autre détecteur de gaz, qui est configuré pour la détermination de la concentration en oxygène comme deuxième composant dans le gaz de mesure (3), et **en ce que** l'unité d'exploitation (8) présente des moyens pour la détermination d'une valeur chiffrée pour la concentration en NOₓ dans le gaz de mesure (3) à partir de la valeur de mesure déterminée avec le premier détecteur de gaz pour la concentration en NOₓ dans le gaz de mesure et du signal de détecteur du deuxième détecteur de gaz, et **en ce qu'**il est prévu un moyen de mémorisation, dans lequel une première courbe caractéristique pour l'exploitation des signaux de détecteur du premier détecteur de gaz et une deuxième courbe caractéristique pour l'évaluation du deuxième signal de détecteur sont mémorisées, dans lequel la première courbe caractéristique (19, 20, 21) est la courbe caractéristique d'un détecteur par chimioluminescence et la deuxième courbe caractéristique représente les écarts de la courbe caractéristique d'un détecteur électrochimique, d'un détecteur infrarouge, d'un détecteur UV ou d'un détecteur à semi-conducteur sensible aux NOₓ par rapport à la première courbe caractéristique pour différentes valeurs de la concentration du deuxième composant dans le gaz de mesure.

11. Dispositif selon la revendication 9 ou 10, **caractérisé en ce que** des moyens sont configurés pour l'introduction (11) et/ou la détermination de la concentration d'un autre composant dans le gaz de mesure, et **en ce que** différentes courbes caractéristiques (19, 20, 21) pour différentes valeurs chiffrées de cette concentration de l'autre composant sont mémorisées, à partir desquelles on peut déduire respectivement la valeur de différence ou le facteur dont la valeur chiffrée s'écarte de la valeur de mesure pour la concentration en NOₓ dans le gaz de mesure.

12. Dispositif selon l'une quelconque des revendications 9 à 11, **caractérisé en ce qu'**un refroidisseur de gaz ou un piège à condensat est agencé dans le flux de gaz du gaz de mesure et/ou **en ce qu'**il est prévu des moyens pour reconnaître la présence d'un refroidisseur de gaz ou d'un piège à condensat dans le flux de gaz du gaz de mesure et/ou des moyens pour reconnaître et/ou introduite la température de travail du refroidisseur de gaz ou du piège à condensat et/ou des moyens pour déterminer la teneur en humidité dans le gaz de mesure, en particulier avant l'entrée dans un refroidisseur de gaz.

13. Dispositif selon l'une quelconque des revendications 9 à 12, **caractérisé en ce que** le deuxième détecteur de gaz est un détecteur électrochimique d'O₂ et/ou le dispositif (2) est réalisé sous forme portable.
